# EUROPÄISCHE PATENTANMELDUNG

(11) **EP 1 700 862 A1**
(43) Veröffentlichungstag der Anmeldung: **13.09.2006**
(21) Anmeldenummer: 06004075.5
(22) Anmeldetag: 28.02.2006
(51) Int. Cl.: C07J 71/00

(54) **Verfahren zur Herstellung von polymorph stabilem Triamcinolonacetonid**

(30) Priorität: 01.03.2005 DE 102005009194
(71) Anmelder: HPP - Hommel Pharmaceuticals Production GmbH, 39179 Barleben (DE)
(72) Erfinder: Völkl, Klaus-Peter, 59348 Lüdinghausen (DE); Näther, Christian, 24106 Kiel (DE)
(74) Vertreter: Linnemann, Winfried

(57) **Zusammenfassung**

Die Erfindung betrifft ein Verfahren zur Herstellung von polymorph stabilem Triamcinolonacetonid. Es soll ein Verfahren zur Herstellung von polymorph stabilem Triamcinolonacetonid, insbesondere zur Verwendung als sterile Suspension in wäßrigen Lösungen zur Behandlung von Entzündungen, geschaffen werden, wobei das Triamcinolonacetonid hergestellt wird durch Auskristallisieren oder Fällen aus mindestens einem Lösungsmittel. Dabei soll ein für eine lange Lagerbarkeit der Suspension ausreichend hoher Anteil der stabilen Modifikation des Triamcinolonacetonids gewährleistet werden.

Das erfindungsgemäße Verfahren sieht vor, daß bei der Kristallisation oder Fällung Wasser oder Methanol oder ein Wasser-Methanol-Gemisch vorhanden ist, daß die Kristallisation oder Fällung in einem Temperaturbereich zwischen -80°C und 200°C und in einem Druckbereich zwischen 10 hPa und 5000 hPa erfolgt und daß dabei das Triamcinolonacetonid zu wenigstens 30 Gew.-% in seiner trigonalen Modifikation hergestellt wird.

Mit diesem Verfahren wird eine lange Lagerbarkeit der Suspension erreicht, weil ein Verfilzen und Verfestigen eines sedimentierten Bodensatzes in der wässerigen Lösung ausgeschlossen wird.

## Beschreibung

Die vorliegende Erfindung betrifft ein Verfahren zur Herstellung von polymorph stabilem Triamcinolonacetonid, insbesondere zur Verwendung als sterile Suspension in wäßrigen Lösungen zur Behandlung von Entzündungen, wobei das Triamcinolonacetonid hergestellt wird durch Auskristallisieren oder Fällen der gelösten Substanz aus mindestens einem Lösungsmittel.

Bei Suspensionen von Triamcinolonacetonid in wässerigen Lösungen ist gelegentlich zu beobachten, daß ein sedimentierter Bodensatz sich nach einer gewissen Zeit verfilzt und sich nicht mehr zu einer Suspension aufschütteln läßt. Dieses Phänomen tritt in der Regel bis zu etwa sechs Monate nach Herstellung der Suspension auf und läuft dann innerhalb von etwa ein bis zwei Tagen ab. Durch dieses Verfilzen des sedimentierten Bodensatzes wird die Suspension unbrauchbar.

Eine Erklärung für die Verfilzung und Verfestigung des Sediments ist die Umwandlung einer instabilen Modifikation in eine stabile Modifikation des Triamcinolonacetonids. Untersuchungen haben gezeigt, daß Triamcinolonacetonid als Suspension in Wasser in nur einer kristallinen Form bei Zimmertemperatur stabil ist. Diese stabile Form wurde als trigonale Modifikation identifiziert.

Für die vorliegende Erfindung stellt sich deshalb die Aufgabe, ein Verfahren zur Herstellung von reinem, kristallinen, sterilen Triamcinolonacetonid anzugeben, mit dem die als Suspension in Wasser bei Zimmertemperatur stabile trigonale Modifikation des Triamcinolonacetonids mit einem ausreichend hohen Anteil an der Gesamtmenge des erzeugten Triamcinolonacetonids erzeugt werden kann, um eine Verfilzung des Sediments zu verhindern und um eine stabile Suspension herzustellen.

Die Lösung dieser Aufgabe gelingt erfindungsgemäß mit einem Verfahren der eingangs genannten Art, das dadurch gekennzeichnet ist,
- daß bei der Kristallisation oder Fällung Wasser oder Methanol oder ein Wasser-Methanol-Gemisch vorhanden ist,
- daß die Kristallisation oder Fällung in einem Temperaturbereich zwischen -80°C und 200°C erfolgt,
- daß die Kristallisation oder Fällung in einem Druckbereich zwischen 10 hPa und 5000 hPa erfolgt und
- daß dabei das Triamcinolonacetonid zu wenigstens 30 Gew.-% in seiner trigonalen Modifikation erzeugt wird.

Mit dem erfindungsgemäßen Verfahren wird vorteilhaft erreicht, daß das hergestellte Triamcinolonacetonid in einem zumindest ausreichenden Mengenanteil in seiner trigonalen Modifikation erzeugt wird. Insbesondere wird somit ein Verfilzen und Verfestigen des sedimentierten Bodensatzes in der wässerigen Lösung ausgeschlossen. Damit wird sichergestellt, daß eine wässerige Suspension von so hergestelltem Triamcinolonacetonid auch unter einfachen Lagerbedingungen, insbesondere bei Raumtemperatur, eine ausreichend lange Haltbarkeit aufweist und daß auch nach längerer Lagerzeit, z.B. über mehrere Monate, sich ein sedimentierter Bodensatz wieder zur Suspension aufschütteln läßt.

In einer Weiterbildung des Verfahrens ist vorgesehen, daß die Kristallisation oder Fällung in einem Temperaturbereich zwischen -50°C und 110°C erfolgt.

Eine weitere bevorzugte Ausgestaltung des Verfahrens sieht vor, daß die Kristallisation oder Fällung in einem Druckbereich zwischen 150 hPa und 1500 hPa erfolgt.

Durch eine geeignete und optimierte Wahl von Temperatur und Druck bei der Kristallisation oder Fällung kann erreicht werden, daß das hergestellte Triamcinolonacetonid auch zu mehr als 30 Gew.-% in seiner stabilen trigonalen Modifikation entsteht. Grundsätzlich ist es dabei möglich, wenn auch nicht unbedingt oder immer erforderlich, die stabile trigonale Modifikation bis zu einem Anteil von 100 Gew.-% herzustellen.

Weiterhin ist für das erfindungsgemäße Verfahren bevorzugt vorgesehen, daß als Lösungsmittel für das Auskristallisieren des Triamcinolonacetonids Aceton, Ethanol, Ethylacetat, Butanol, Isopropanol, n-Propanol, Methylethylketon oder Dimethylformamid verwendet wird. Die genannten Lösungsmittel sind gut für ein Auskristallisieren mit vertretbarem Energieaufwand geeignet und gewährleisten gleichzeitig, daß die Herstellung der gewünschten stabilen trigonalen Modifikation des Triamcinolonacetonids nicht negativ beeinflußt wird.

Für die Ausführungen des Verfahrens, bei denen das Triamcinolonacetonid durch Fällen hergestellt wird, ist bevorzugt vorgesehen, daß das Fällen der gelösten Substanz durch Zusatz eines Lösungsmittels mit schlechterer Löslichkeit zu einer Lösung der Substanz mit einer besseren Löslichkeit erfolgt.

Bevorzugt ist dabei in konkreter Ausgestaltung vorgesehen, daß als Lösungsmittel mit schlechterer Löslichkeit Wasser und als Lösungsmittel mit besserer Löslichkeit eine gesättigte acetonische Lösung verwendet wird. Die beiden hier verwendeten Lösungsmittel sind gängig und einfach und sicher handhabbar, was zu einer wirtschaftlichen Ausführung des Verfahrens beiträgt.

Eine weitere Ausgestaltung des erfindungsgemäßen Verfahrens sieht vor, daß die Temperatur und der Druck während der Kristallisation oder Fällung konstant gehalten werden. Bei konstanter Temperatur und konstantem Druck läßt sich das Verfahren verhältnismäßig einfach ausführen, wobei aber schon gewährleistet ist, daß die gewünschte stabile trigonale Modifikation des Triamcinolonacetonids mit einem ausreichenden Mengenanteil hergestellt wird.

Eine alternative Ausgestaltung des erfindungsgemäßen Verfahrens schlägt vor, daß die Temperatur und/oder der Druck während der Kristallisation oder Fällung zeitabhängig variiert werden/wird. Durch die zeitabhängige Variation der Temperatur und/oder des Drucks kann der Ablauf der Kristallisation oder Fällung so optimiert werden, daß eine höhere Ausbeute der gewünschten stabilen trigonalen Modifikation des Triamcinolonacetonids erreicht wird, wobei im optimalen Fall die Ausbeute der trigonalen Modifikation bis zu 100 % betragen kann.

Für Suspensionen des erfindungsgemäß hergestellten Triamcinolonacetonids in wässerigen Lösungen wird schon bei einem Anteil der stabilen trigonalen Modifikation des Triamcinolonacetonids von wenigstens 30 Gew.-%, vorzugsweise von wenigstens 50 Gew.-%, bei üblichen Lagerungsbedingen für Arzneimittel bei Raumtemperatur schon eine ausreichend lange und praxisgerechte Haltbarkeit erreicht. Für erschwerte Lagerbedingungen, z. B.. bei erhöhter Temperatur, kann es zweckmäßig sein, einen höheren Anteil der trigonalen Modifikation des Triamcinolonacetonids in der Suspension zu haben, wobei der Anteil bis zu 100 Gew.-% betragen kann. In diesem Falle ist dann auch unter sehr schwierigen und ungünstigen Lagerbedingungen eine lange Haltbarkeit ohne Verfestigung und Verfilzung des sedimentierten Bodensatzes der Suspension gewährleistet.

## Patentansprüche

1. Verfahren zur Herstellung von polymorph stabilem Triamcinolonacetonid, insbesondere zur Verwendung als sterile Suspension in wäßrigen Lösungen zur Behandlung von Entzündungen, wobei das Triamcinolonacetonid hergestellt wird durch Auskristallisieren oder Fällen der gelösten Substanz aus mindestens einem Lösungsmittel,
**dadurch gekennzeichnet ,**
- **daß** bei der Kristallisation oder Fällung Wasser oder Methanol oder ein Wasser-Methanol-Gemisch vorhanden ist,
- **daß** die Kristallisation oder Fällung in einem Temperaturbereich zwischen -80°C und 200°C erfolgt,
- **daß** die Kristallisation oder Fällung in einem Druckbereich zwischen 10 hPa und 5000 hPa erfolgt und
- **daß** dabei das Triamcinolonacetonid zu wenigstens 30 Gew.-% in seiner trigonalen Modifikation hergestellt wird.

2. Verfahren nach Anspruch 1, **dadurch gekennzeichnet, daß** die Kristallisation oder Fällung in einem Temperaturbereich zwischen -50°C und 110°C erfolgt.

3. Verfahren nach Anspruch 1 oder 2, **dadurch gekennzeichnet, daß** die Kristallisation oder Fällung in einem Druckbereich zwischen 150 hPa und 1500 hPa erfolgt.

4. Verfahren nach einem der vorangehenden Ansprüche, **dadurch gekennzeichnet, daß** als Lösungsmittel für das Auskristallisieren des Triamcinolonacetonids Aceton, Ethanol, Ethylacetat, Butanol, Isopropanol, n-Propanol, Methylethylketon oder Dimethylformamid verwendet wird.

5. Verfahren nach einem der vorangehenden Ansprüche, **dadurch gekennzeichnet, daß** das Fällen der gelösten Substanz durch Zusatz eines Lösungsmittels mit schlechterer Löslichkeit zu einer Lösung der Substanz mit einer besseren Löslichkeit erfolgt.

6. Verfahren nach Anspruch 5, **dadurch gekennzeichnet, daß** als Lösungsmittel mit schlechterer Löslichkeit Wasser und als Lösungsmittel mit besserer Löslichkeit eine gesättigte acetonische Lösung verwendet wird.

7. Verfahren nach einem der Ansprüche 1 bis 6, **dadurch gekennzeichnet, daß** die Temperatur und der Druck während der Kristallisation oder Fällung konstant gehalten werden.

8. Verfahren nach einem der Ansprüche 1 bis 6, **dadurch gekennzeichnet, daß** die Temperatur und/oder der Druck während der Kristallisation oder Fällung zeitabhängig variiert werden/wird.
